# EUROPEAN PATENT APPLICATION

(11) **EP 2 380 578 A1**
(43) Date of publication of application: **26.10.2011**
(21) Application number: 09834449.2
(22) Date of filing: 22.12.2009
(51) Int. Cl.: A61K 33/26, A61K 31/122, A61K 31/355, A61K 31/375, A61K 47/02, A61K 47/08, A61K 47/22, A61P 1/00, A61P 1/04, A61P 1/16, A61P 3/02, A61P 3/10, A61P 9/12, A61P 13/12, A61P 15/00, A61P 17/00, A61P 17/14, A61P 29/00, A61P 31/04, A61P 35/00, A61P 37/08

(54) **BIOACTIVE AGENT, PHARMACEUTICAL PRODUCT, COSMETIC PRODUCT, FRESHNESS KEEPING AGENT, AND PLANT AND ANIMAL GROWTH PROMOTING AGENT**

(30) Priority: 25.12.2008 JP 2008330569
(71) Applicant: I.B.E. Co., Ltd., Aichi 444-0703 (JP)
(72) Inventor: MAKINO, Shinji, Nishio-shi, Aichi 444-0703 (JP)
(74) Representative: McCluskie, Gail Wilson
(86) International application number: PCT/JP2009/007151
(87) International publication number: WO 2010/073642

(57) **Abstract**

The subject of the present invention is to provide a bioactivator which is useful as a medicine, growth promoting agent for plants and animals, and the like, wherein the bioactive effectiveness of a ferric salt is reinforced and stabilized. One or more kind of vitamin selected from a group consisting of vitamins C, E and K is (are) added to a ferric salt as an anti-oxidizing and bioactivity reinforcing agent, with a magnesium salt being further added as a stabilizer.

## Description

### FIELD OF THE INVENTION

The present invention relates to a bioactivator useful as medicine, cosmetics, a freshness keeping agent, growth promoting agent for animals and plants, and the like.

### BACKGROUND OF THE INVENTION

For instance, an iron salt such as ferrous iron salt, ferric iron salt, and ferric ferrous iron salt, is bioactive and known to be useful as medicine, cosmetics, a freshness keeping agent, growth promoting agent for animals and plants, and the like. For instance, water containing a ferric ferrous iron salt is well known as π-water.

Patent Literature 1: Tokkai 2002-80376

### DISCLOSURE OF THE INVENTION

### PROBLEMS TO BE SOLVED BY THE INVENTION

Nevertheless, there is a problem in that said iron salt is apt to be oxidized, making the bioactive ability of said iron salt unstable, so that the effect of said iron salt will deteriorates over a long period of preservation.

### MEANS TO SOLVE SAID PROBLEM

To solve said problem, the present invention selects a ferric salt having the largest bioactive effectiveness among iron salts, and provides a bioactivator, to which one or more kinds of vitamin having been selected from a group consisting of vitamins C, E, and K is (are) added as an anti-oxidizing and bioactivity reinforcing agent, with magnesium salt(s) being further added as a stabilizer.
It is desirable that said ferric salt(s) and said vitamin(s) be mixed together at a molar ratio in the range of between 1:1 to 1:10⁶, and that said ferric salt(s) and said magnesium salt(s) be mixed together at a molar ratio in the range of between 1:1 to 1:10⁶.
Further, the present invention provides medicines for the treatment of diabetes, hypertension, cancer, hepatitis, rheumatism, atopic dermatitis, and the like, said treatment medicines being made from said bioactivator.

### EFFECTS OF THE INVENTION

The oxidation of a ferric salt, which is useful as a bioactivator, is prevented by vitamins C, E and K having anti-oxidation properties, while at the same time the bioactivity of said ferric salt is reinforced by said vitamins, with the reinforced bioactivity of said ferric salt being further stabilized by s magnesium salt, so that the present invention provides medicine, cosmetics, a freshness keeping agent, growth promoting agent for animals and plants, having durability for long preservation.

### PREFERRED EMBODIMENT TO EXECUTE THE INVENTION

The present invention is explained precisely hereafter.

### [Ferric iron salt]

The ferric iron salts, used as bioactivators in this invention, include inorganic acid salts such as hydrochloride, sulfate, nitrate, phosphate and the like, organic acid salts such as acetate, formate, oxalate, citrate, lactate, butyrate, succinate, propionate, and the like. Two or more kinds of ferric iron salt may be used together.

### [Vitamins]

The vitamins used in the present invention as anti-oxidants for said ferric salts are vitamins C, E and K.
Said vitamin C is an L-ascorbic acid, and an alkalimetal salt of said L-ascorbic acid is usable in the present invention. A desirable alkali metal salt of said L-ascorbic acid is L-potassium ascorbate. In the present invention, both L-ascorbic acid and the alkalimetal salt of L-ascorbic acid are defined as vitamin C.
Said vitamin E is tocopherol, and said tocopherol includes α-tocopherol, β-tocopherol, γ-tocopherol, δ-tocopherol, ε-tocopherol, ζ-tocopherol, η-tocopherol, and the like. α-tocopherol being the most effective.
Said vitamin K includes phylloquinone (vitamin K₁), menaquinone (vitamin K₂), menadion (vitamin K₃), and the like.
Two or more kinds of said vitamin may be used together. The most desirable vitamin of said vitamins is vitamin C, which has the best anti-oxidizing and bioactivity reinforcing properties for said ferric salts.

[Magnesium salt]
The magnesium salts, used in the present invention, include inorganic acid salts such as magnesium chloride, magnesium sulfate, magnesium phosphate, magnesium nitrate, and the like, organic acid salts such as magnesium acetate, magnesium butyrate, magnesium format, magnesium oxalate, magnesium citrate, magnesium propionate, and the like. Two or more kinds of magnesium salt may be used together.
To stabilize the bioactivity of said ferric salt, said magnesium salt is preferably added in a molar amount equal to, or more than that of said ferric salt.
In a case where said magnesium salt is added in an amount beyond 10⁶ mole per 1 mole of said ferric salt, an excessively large amount of water may be necessary to dissolve said magnesium salt uniformly in the water, resulting in the possibility of the concentration of said ferric salt being low.

### [Preparation]

Commonly, said iron salt and magnesium salt are each prepared as an aqueous solution, and vitamins E and K, both being fat-soluble, are dispersed and emulsified in water, into which a surfactant has been dissolved, to prepare a dispersion or an emulsion.
In said aqueous solution, dispersion, or emulsion, said ferric salt and vitamins are commonly mixed together in a molar ratio in the range of between 1:1 to 1:10⁶, desirably 1:1 to 1:10⁴, more desirably 1:1 to 1:10², while said ferric salt and magnesium salt are commonly mixed together in a molar ratio in the range of between 1:1 to 1:10⁶.
To obtain a remarkable anti-oxidant effect for said ferric salt, said vitamins are preferably added in a molar amount equal to, or more than that of said ferric salt, but in a case where said vitamins are added in an amount beyond 10⁶ mole per 1 mole of said ferric salt, said vitamins will be difficult to dissolve or disperse uniformly in said bioactivator, leading to concerns that excess amount of vitamins will have harmful effect on the bioactivity of said ferric salt.
To said aqueous solution, dispersion, or emulsion, further vitamins, with the exception of vitamins C, E and K, hormones, fats and oils, humectants, perfumery spices, sweetenings, or the like may be added..

The bioactivator of the present invention is mainly administered orally, or by mixing in with food as is, further said bioactivator can also be administered by injection, instillation, or percutaneously.

Said bioactivator of the present invention is especially useful for the treatment or prevention of cancer, diabetes, hepatitis, nephritis, renal failure, gastric ulcer, duodenal ulcer, hypertension, collagen disease, allergic diseases such as atopic dermatitis, pollinosis, and the like, menorrhagia, obstipation, and the like, and further useful as an antimicrobial agent.
Further, said bioactivator of the present invention is useful as cosmetics, since said bioactivator has a beautificative effect on skin as well as being a preventive or treatment for dermatitis, and further said bioactivator promotes the growth of animals and plants, and works as a flavor enhancer.

### [Example 1] (Preparation of bioactivator 1)

One quarter mole of Fecl₂ anhydride, 2.5 moles of vitamin C (potassium L-ascorbate) and 3 moles of Mgcl₂ anhydride were dissolved in water to be 1 liter in total amount, and the resulting aqueous solution was further diluted 100 times with water, to prepare the original solution of bioactivator 1.

### [Example 2] (Preparation of bioactivator 2)

Five percent by mass of α-tocophenol was dispersed in water into which 1% by mass of a fatty acid diglyceride had been dissolved, to prepare a vitamin E aqueous dispersion.
The resulting aqueous dispersion was then added to an aqueous solution, into which 0.3 mole of Fecl₄ anhydride and 30 moles of Mgcl₂ anhydride had been dissolved in water, so that the amount of α-tocophenol in the resulting aqueous solution was to be 60 moles, and then the total amount of the resulting aqueous solution was adjusted to be 1 liter by diluting it with water, and further the resulting aqueous solution was then further diluted 100 times with water, to prepare the original solution of bioactivator 2.

### [Example 3] (Preparation of bioactivator 3)

Five percemt by mass of vitamin K₃ was dispersed in water into which 1.5% by mass of a sorbitan fatty acid ester had been dissolved, to prepare vitamin K₃ aqueous dispersion.
The resulting aqueous dispersion was then added to an aqueous solution, in which 0.27 mole of FeCl₂ anhydride and 54 moles of MgSO₄ anhydride had been dissolved in water, so that the amount of vitamin K3 in the resulting aqueous solution was to be 108 moles, and then the total amount of the resulting aqueous solution was then adjusted to be 1 liter by diluting it with water, and then the resulting aqueous solution was further diluted 100 times with water, to prepare the original solution of bioativator 3.

### [Example 4] (Preparation of bioactivator 4)

One quarter mole of sodium L-ascorbate, instead of the potassium L-ascorbate used in Example 1, was used in the Example, and further 0.25 mole of FeCl₂ anhydride and 0.25 mole of MgCl₂ anhydride were dissolved in water, and the total amount of the resulting aqueous solution was adjusted to be 1 liter, and then the resulting aqueous solution was diluted 100 times with water, to prepare the original solution of bioactivator 4.

### [Reference 1] (Preparation of bioactivator 1A)

Two point five moles of vitamin C (potassium L-ascorbate) was added and dissolved in an aqueous solution, into which 0.2 mole of FeCl₂ anhydride and 3 moles of MgCl₂ anhydride had been dissolved, then the total amount of the resulting aqueous solution was adjusted to be 1 litler, and the resulting aqueous solution was then further diluted 100 times with water, to prepare the original bioactivator 1A.

### [Reference 2] (Preparation of bioactivator 2A)

The vitamin E aqueous dispersion prepared in Example 2 was added and dissolved in an aqueous solution, into which 0.3 mole of FeCl₄ anhydride had been dissolved, so that the amount of α-tocophenol in the resulting aqueous solution was to be 60 moles, and further 30 moles of MgCl₂ anhydride was then added, and the total amount of the resulting aqueous solution was adjusted to be 1 liter with water and the resulting aqueous solution was then further diluted 100 times with water, to prepare the original solution of bioactivator 2A.

### [Reference 3] (Preparation of bioactivator 3A)

The vitamin K₃ aqueous dispersion prepared in Example 3 was added to an aqueous solution, into which 0.27 mole of FeCl₂ anhydride had been dissolved, so that the amount of vitamin K3 in the resulting aqueous solution was to be 108 moles, and 54 moles of MgSO₄ anhydride was further added to the resulting aqueous solution, and then the total amount of the resulting aqueous solution was adjusted to be 1 liter with water, and the resulting aqueous solution was then further diluted 100 times, to prepare the original solution of bioactivator 3A.

### [Reference 4] (Preparation of bioactivator 4A)

One quarter mole of sodium L-ascorbate, instead of the potassium L-ascorbate used in Reference 1 was used, and 0.25 mole of sodium L-ascorbate was added to an aqueous solution, into which 0.25 mole of FeCl₂, anhydride and 0.25 mole of MgCl₂ anhydride had been dissolved in water, and the total amount of the resulting aqueous solution was then adjusted to be 1 liter with water, and the resulting aqueous solution was then further diluted 100 times with water, to prepare the original solution of bioactivator 4A.

### [Example 5] (Freshness maintenance test)

Each original solution of the bioactivator 1 to 4, and 1A to 4.A was stored for 6 months after preparation, after which 1 ml of each original solution was diluted with water respectively to be 1 liter and prepare the test solution. Slices of a flatfish were dipped in said solutions respectively, after which said solutions were removed from said slices with a filter paper. Said slices were then wrapped in a polyvinylidene chloride film, and kept at 5°C. After 15 days preservation, the K value in each case was lower than 30%, as shown in Table 1, so that each slice of said flatfish was in the condition wherein said slice can be eaten raw.
Further, referring to Table 1, it was recognized that the bioactivators 1A, 2, 3 and 4A, which were each prepared by dissolving a ferric salt and magnesium salt in water to prepare an aqueous solution and then adding vitamins to said aqueous solution, each had more remarkable bioactivity than the bioactivators 1, 2A, 3A and 4, which were prepared by dissolving a ferric salt and adding vitamins in/to water to prepare an aqueous solution and then adding a magnesium salt thereto.

**[Table 1]**

| | | | | | | | | |
|---|---|---|---|---|---|---|---|---|
| Bioactivator | 1 | 2 | 3 | 4 | 1A | 2A | 3A | 4A |
| K value of slice (%) | 25 | 27 | 28 | 26 | 20 | 29 | 30 | 21 |

[COMPARISON 1]
In COMPARISON 1, the freshness maintenance test as described in EXAMPLE 5 was carried out using a test solution wherein vitamin C and MgCl₂ were added in a molar amount equal to or less than that of FeCl₂. That is to say, 0.25 mole of FeCl₂ anhydride, 0.2 mole of vitamin C, and 0.2 mole of MgCl₂ anhydride were each dissolved in water to prepare 1 liter of an aqueous solution, and the resulting aqueous solution was further diluted 100 times with water, to prepare the original comparison solution 1. After preparation, said original comparison solution 1 was kept for 6 months, after which 1 ml of said original comparison solution 1 was diluted with water to be 1 liter and prepare a comparison test solution 1. A slice of flat fish was then dipped into said comparison test solution 1 in the same way as in Example 1, after which, the solution was removed from said slice with a filter paper. Said slice was then wrapped in a polyvinylidene chloride film and kept at 5°C. Its K value after 15 days of preservation was 45%, and said slice was in a condition wherein said slice is barely edible.

[COMPARISON 2]
In COMPARISON 2, the freshness maintenance test was carried out using a test solution wherein only MgCl₂ was added to FeCl₂. That is to say, 0.25 mole of FeCl₂ anhydride and 3 mole of MgCl₂ anhydride were each dissolved in water to be 1 liter of aqueous solution, and the resulting aqueous solution was then further diluted 100 times with water, to prepare the original comparison solution 2.
After preparation, said original comparison solution 2 was kept for 6 months, after which 1 ml of said original comparison solution 2 was then further diluted with water to be 1 liter and prepare the comparison test solution 2. A slice of flat fish was dipped into said comparison test solution 2 in the same way as in the aforementioned Example, after which said solution was removed from said slice with a filter paper. Said slice was then wrapped in a polyvinylchloride film and kept at 5°C. Its K value after 15 days of preservation was 38%, and said slice was in a condition wherein said slice was not suitable for sashimi, but could be eaten as a cooked fish meat.

[COMPARISON 3]
In COMPARISON 3, the same preservation test was carried out using slice of the flatfish which had been dipped into water. Its K value, after having been stored for 15 days, was about 75%, and said slices of flatfish were inedible. From the results of Example 5 and Comparisons 1 and 2, it was recognized that FeCl₂ retains a sufficient effect to maintain freshness by adding vitamins and MgCl₂, even after six months of preservation.

[Example 6]
One milliliter of the original solutions of said bioactivators 1 to 4 and 1A to 4A, having been preserved for one year after preparation, were diluted with water to be 1 liter and prepare the test solutions. Using said test solutions, pumpkins, potatoes and onions are each cultivated. The conditions of said harvested vegetables are described below.

[Pumpkins]
Appearance: The pumpkins, cultivated using any of the test solutions 1 to 4, and 1A to 4A, each have glossy appearances, and in particular, the pumpkins, cultivated using test solutions 1A, 2, 3, and 4A, each have a thick bright orange colored pulp, and contain twice or more the amount of carotene as compared with ordinary ones.
Taste: Soft and crumbly and sweet. Said pumpkin has a very high sugar content in the range of between 8 and 11 degrees (generally 7 degrees), as shown in Table 2.

**[Table 2]**

| | | | | | | | | |
|---|---|---|---|---|---|---|---|---|
| Bioactivator | 1 | 2 | 3 | 4 | 1A | 2A | 3A | 4A |
| Sugar content | 8 | 11 | 10 | 8 | 10 | 8 | 8 | 11 |

[Potatoes]
Appearance : Potatoes, cultivated using any of the test solutions 1 to 4, and 1A to 4A, have white skins, and in particular the potatoes, cultivated using said test solutions 1A, 2, 3, and 4A, have fewer shoots on their surface. Starch: As shown in Table 3, a high content of more than 18% (generally 16%)
was confirmed regarding all samples.
Vitamin C: As shown in Table 3, a high content of more than 32 mg/100 g
(generally 23 mg/100 g) was confirmed regarding all samples.
Taste: Perfect, being soft and fluffy, and easily crushed in the mouth, and having the special smell and body of potato. Further, said potatoes are suitable for salad use, since said potatoes have little harshness.

**[Table 3]**

| | | | | | | | | |
|---|---|---|---|---|---|---|---|---|
| Bioactivator | 1 | 2 | 3 | 4 | 1A | 2A | 3A | 4A |
| Starch (%). | 18.1 | 18.7 | 18.8 | 18.3 | 18.8 | 18.3 | 18.2 | 19.9 |
| Vitamin C (mg/100g) | 28 | 33 | 32 | 29 | 32 | 26 | 28 | 33 |

[Onion]
Appearance: Each onion cultivated using any of the test solutions 1 to 4, and 1A to 4A, has a glossy appearance and uniform size. Their skin can easily be peeled, their pulp is tight and firm, and they are long term storable. It was recognized by an electron microscope, or the like that each onion was from a healthy crop having tissue in which their small cells are packed closely. Taste: Said onions are easily cut with a kitchen knife, and taste good enough to eat raw. Since the sugar degree of sugar content of each onion is higher than 8 degrees, much higher than the ordinary 6 degrees, said onions have a sweetness, and a crunchiness when bitten, making said onions suitable for salad, and firm enough for stir-frying.

**[Table 4]**

| | | | | | | | | |
|---|---|---|---|---|---|---|---|---|
| Bioactivator | 1 | 2 | 3 | 4 | 1A | 2A | 3A | 4A |
| Sugar content degree | 8.5 | 10 | 10 | 9.0 | 10.5 | 8.7 | 9.0 | 10 |

[Example 7] (Medical efficacy)
In a case where the original solution of said bioactivator 1 prepared in Example 1 is used as a medicine, generally the following drinking method is applied.
(1) The following quantity of the original solution of said bioactivator 1 is added to a cup of water (in an amount of about 150 ml), mixed well and then said diluted solution is to be drunk three times a day, when getting up, before lunch, and prior to going to bed.
(2) Drinking quantity
   First week: 3 drops X 3 times a day (9 drops in a day)
   Second week: 5 drops X 3 times a day (15 drops in a day)
   Third week: 10 drops X 3 times a day (30 drops in a day)
   Fourth week: 20 drops X 3 times a day (60 drops in a day)
(3) Drinking quantity after fifth week
   a. Cancer: 30 drops X 3 times a day (90 drops in a day)
   b. Diabetes, Hepatitis, Gastric ulcer, Heart disease, Asthma, Hypertension, etc.: 20 drops X 3 times a day (60 drops in a day)
   c. Renal insufficiency, Rheumatism, Atopic dermatitis, Pollinosis, etc.: 10 drops X 3 times a day (30 drops in a day)
   d. Menorrhagia, Obstipasion, Sickness from drinking, other minor diseases: 10 drops X once a day (10 drops in a day)
   e. Maintenance of health: 3 drops X 3 times a day (9 drops in a day)
The results in a case where said bioactivator 3 was administered to patients having various diseases, according to the aforementioned drinking methods, are shown in Tables 5 to 15. In any of said Tables, the numerical values of the test data in the upper rows show the pre-administration numerical values, while the numerical values of the test data in the lower rows show the post-administration numerical values.

**Table 5**

| cases | name of disease | patients | | inspection data | | | observation |
|---|---|---|---|---|---|---|---|
| | | sex | age | blood sugar level *1 | HbAlc *2 | neutral fat *3 | |
| 1 | diabetes | male | 65 | 324 | 9.4 | 341 | Numerical values were improved after drinking for three months, as shown in Table. |
| | | | | 186 | 7.8 | 215 | |
| 2 | diabetes | female | 62 | 370 | 10.2 | 254 | Numerical values were improved after drinking for three months, as shown in Table. |
| | | | | 273 | 9.0 | 132 | |
| 3 | diabetes | male | 42 | 524 | 12.8 | | At the start of drinking, having been taking 20 units of insulin, and 2 tablets of the blood sugar descending agent, one tablet before breakfast, and one tablet before supper, but stopped taking them after drinking for three months. |
| | | | | 108 | 5.6 | 132 | |
| 4 | diabetes | female | 54 | 320 | 9.0 | 227 | Numerical values were improved after drinking for three months, as shown in Table. |
| | | | | 146 | 6.8 | 121 | |

| | | | | | | | |
|---|---|---|---|---|---|---|---|
| *1 the blood sugar level: normal values 70-110 ml/dl *2 HbAlc: normal values 4.0-6.0% *3 neutral fat (triglyceride): normal values 50-140 mg/dl | | | | | | | |

**Table 6**

| cases | name of disease | patients | | inspection data | observation |
|---|---|---|---|---|---|
| | | sex | age | blood pressure *1 | |
| 1 | high blood pressure | female | 53 | 190/115 | Numerical values stabilized after for month. |
| | | | | 151/71 | drinking one |
| 2 | high blood pressure and cerebrovascular infarction | female | 48 | 135/96 | Blood pressure had barely been kept at 135/96 as a result of taking the blood pressure descending agents, but after drinking for three |
| | | | | 120/84 | months, could stop taking, and after drinking for five months, numerical values were improved, as shown in Table. |
| 3 | diabetes | male | 65 | 181/120 | Subjective symptoms vanished completely after drinking for three |
| | | | | 140/86 | months, and numerical values were improved, as shown in Table. |

| | | | | | |
|---|---|---|---|---|---|
| *1 blood pressure: normal values 139-101/89-61 mmHg | | | | | |

**Table 7**

| case | name of disease | patient | | inspection data | | | | | | observation |
|---|---|---|---|---|---|---|---|---|---|---|
| | | sex | age | RBC *1 | WBC *2 | Hb *3 | Ht *4 | BUN *5 | CRP *6 | |
| 1 | systemic lupus erythematosus | female | 32 | 3.3 million | 9200 | 7.4 | 23.5 | 32 | 4 | Normal numerical values were improved after drinking for 12 months, as shown Table. |
| | | | | 4.2 million | 6500 | 12.4 | 37.4 | 17.4 | 0.6 | |

| | | | | | | | | | | |
|---|---|---|---|---|---|---|---|---|---|---|
| *1 RBC = erythrocyte (red) count(red blood corpuscles): normal values 3.5 million-4.5 million/mm³ *2 WBC = leukocyte (white) count: normal values 4000-9000/mm³ *3 Hb = hemoglobin: normal values 2-15 g/dl *4 Ht = hematocrit: normal values 36-45% (adult female) *5 BUN = blood urea nitrogen: normal values 8∼20mg/dl *6 CRP: normal values less than 1.0mg/dl | | | | | | | | | | |

**Table 8**

| cases | name of disease | patients | | inspection data | | | | | observation |
|---|---|---|---|---|---|---|---|---|---|
| | | sex | age | GOT *1 | GPT *2 | γ-GTP *3 | tumor marker AFP *4 | tumor marker TPA *5 | |
| 1 | cancer of liver | male | 64 | 59 | 64 | 190.3 | 47.6 | 287 | Since he was diagnosed with liver cancer three year ago, he had been treated with and anticancer drug. Numerical values were improved after drinking for three months, as shown in Table. |
| | | | | 46 | 29 | 79.2 | 18.4 | 98 | |
| 2 | cancer of liver | male | 55 | 128 | 76 | 214.6 | 54.1 | 316 | Progressing from viral hepatitis type C→Cirrhosis→liver cancer, but the condition was improved and his numerically valued tumor marker also showed improvement after drinking for six months as shown in Table. |
| | | | | 59 | 42 | 89.2 | 18.9 | 99 | |

| | | | | | | | | | |
|---|---|---|---|---|---|---|---|---|---|
| *1 GOT: normal values 5-35KU/ml *2 GPT: nomal values 5∼25 KU/ml *3 γ-GTP: normal values less than 40 units (adult) *4 tumor marker AFP: normal values less than20 ng/ml (RIA) *5 tumor marker TPA: normal values less than 110 U/l (RIA) | | | | | | | | | |

**Table 9**

| cases | name of disease | patients | | inspection data | | | | observation |
|---|---|---|---|---|---|---|---|---|
| | | sex | age | tumor marker PAP *1 | tumor marker PSA *2 | CA125 *3 | CA19-9 *4 | |
| 1 | cancer of the prostate | male | 48 | 218 | | | | Numerical values were improved by drinking for three months, as shown in Table. |
| | | | | 0.7 | | | | |
| 2 | cancer of the prostate | male | 62 | | 4.1 | | | Complete recovery by drinking for two months. |
| | | | | | 1.7 | | | |
| 3 | ovarian cancer | female | 60 | | | 280 | | A tumor having a size of about 5 cm had reduced to about 1 cm after drinking for six months, and numerical values were improved after one year, as shown in Table. |
| | | | | | | 36 | | |
| 4 | cancer of the colon | female | 36 | | | | 44.5 | Numerical values were improved by drinking for three months, as shown in Table. |
| | | | | | | | 34.1 | |

**Table 10**

| cases | name of disease | patients | | inspection data | | | | | | observation |
|---|---|---|---|---|---|---|---|---|---|---|
| | | sex | age | WBC *1 | white blood corpuscles *2 | blood platelets *3 | protein M *4 | CA125 *5 | CA19-9 *6 | |
| 1 | acute myelocytic leukemia | female | 48 | 1900 | | | | | | Headache, stiff shoulders, nausea, back muscleache, constipation, halitosis and the like wholly vanished, and physical condition was also improved after drinking for three months. |
| | | | | 3900 | | | | | | |
| 2 | myelocytic leukemia | male | 62 | | 9600 | 6300 | | | | Numerical values were improved after drinking for 10 days, as shown in Table. |
| | | | | | 5600 | 185000 | | | | |
| 3 | multiple myeloma | female | 60 | | 2600 | 80000 | 9050 | | | Numerical values of leukocyte and thrombocyte were improved to reach certainly normal numerical values, the numerical although present values were still rather lower, after drinking for three months, than normal numerical values. |
| | | | | | 3500 | 120000 | 2210 | | | |
| 4 | hypoplastic anemia | female | 36 | | | | | 2100 | 970 | First, by inspection, it was doubtful that this anemia was malignant, but numerical values were improved after drinking for three months, as shown in Table, confirming that this anemia was benign. |
| | | | | | | | | 40 | 78 | |

| | | | | | | | | | | |
|---|---|---|---|---|---|---|---|---|---|---|
| *1 WBC: normal values 4000-9000 /mm³ *2 white blood corpuscles: normal values 4000-9000 /mm³ *3 blood platelets: normal values 0.2 million-0.4 million/n *4 protein M: normal values less than 1700/mm³ *5 CA125: normal values less than 50 U/ml *6 CA19-9: normal values less than 37 U/ml | | | | | | | | | | |

**Table 11**

| cases | name of disease | patients | | inspection data | | | | observation |
|---|---|---|---|---|---|---|---|---|
| | | sex | age | GOT *1 | GPT *2 | γ-GTP *3 | ZTT *4 | |
| 1 | hepatitis | female | 56 | 81 | 105 | | | Two kinds of herbal medicine were taken to no effect, but improvement was noted after drinking for one month. |
| | | | | 35 | 38 | | | |
| 2 | chronic viral hepatitis type B | male | 52 | 79 | 136 | | | Numerical values were completely improved after drinking for three months. |
| | | | | 31 | 20 | | | |
| 3 | viral hepatitis type B | male | 49 | 88 | 42 | | | Numerical values were completely improved after drinking for one month. |
| | | | | 32 | 17 | | | |
| 4 | viral hepatitis type C | female | 47 | 91 | 162 | 94 | 15 | Liver functions began to improve by drinking for one month. |
| | | | | 42 | 86 | 61 | 10.6 | |
| 5 | chronic viral hepatitis type C | male | 67 | 91 | 180 | | | Quantitative-qualitative analysis reaction of the antigen of virus type C hepatitis became negative by drinking for one year. |
| | | | | 19 | 17 | | | |

| | | | | | | | | |
|---|---|---|---|---|---|---|---|---|
| *1 GOT: normal values 5-35 KU/ml *2 GPT: normal values 5-25 KU/ml *3 γ-GTP: normal values less than 40 units (adult) *4 ZZT: normal values 2-14 units | | | | | | | | |

**Table 12**

| cases | name of disease | patients | | inspection data | | observation |
|---|---|---|---|---|---|---|
| | | sex | age | CRP *1 | RF *2 | |
| 1 | multiple articular rheumatism | female | 68 | 1.8 | 112 | Numerical values were improved by drinking for three months, as shown in Table. |
| | | | | 0.6 | 47 | |
| 2 | rheumatism | male | 45 | 2.1 | 81 | Numerical values were improved with swelling and aching of also after for fingers showing improvement drinking three months, as shown in Table. |
| | | | | 1.0 | 37 | |

| | | | | | | |
|---|---|---|---|---|---|---|
| *1 1 CRP: normal values less than 1.0 mng/dl *2 RF = rheumatoid factors: normal values less than 35 U/ml | | | | | | |

**Table 13**

| cases | name of disease | patients | | inspection data | | | | | observation |
|---|---|---|---|---|---|---|---|---|---|
| | | sex | age | Ige-RIST *1 | cat *2 | cedar *3 | house dust *4 | weeds *5 | |
| 1 | atopic dermatitis | male | 37 | 4156 | 11.63 | 40.26 | ≧ 100 | 3.23 | Numerical values were improved after drinking for four months, as shown in Table, while at the same time, the taking of steroid medication became unnecessary. |
| | | | | 720 | 8.4 | 30.4 | 60 | 2.2 | |
| 2 | atopic dermatitis | male | 23 | 1671 | | | | | Conditions seemed more improved than the improved numericla values indicated, by drinking for five months. |
| | | | | 1270 | | | | | |

| | | | | | | | | | |
|---|---|---|---|---|---|---|---|---|---|
| *1 Ige-RIST: normal values less than 280 IU/ml *2 cat: normal values less than 0.34 UA/ml *3 cedar: normal values less than 0.34 UA/ml *4 house dust: normal values less than 0.34 UA/ml *5 weeds: normal values less than 0.34 UA/ml | | | | | | | | | |

**Table 14**

| case | name of disease | patient | | inspection data | observation |
|---|---|---|---|---|---|
| | | sex | age | MRSA*1 | |
| 1 | MRSA | female | 78 | positive | Methicillin-Resistant Staphylococcus Aureus (MRSA) positive changed to MRSA negative by drinking for three months. |
| | | | | negative | |

| | | | | | |
|---|---|---|---|---|---|
| *1 MRSA: normal values negative | | | | | |

**Table 15**

| cases | name of disease | patients | | inspection data | | | | observation |
|---|---|---|---|---|---|---|---|---|
| | | sex | age | triglyceride*1 | obesity index*2 | γ-GTP *3 | amount of urine*4 | |
| 1 | obesity | female | 57 | 230 | +17.2% | | | Numerical values were improved by drinking for three months, as shown in Table. |
| | | | | 165 | +9.4% | | | |
| 2 | emaciation slight hepatopathy | and female | 48 | | -16.2% | 86 | | Numerical values were improved by drinking for three months, as shown in Table. |
| | | | | | -7.1% | 44 | | |
| 3 | chronic renal failure | male | 45 | | | | 40-90 | Quantity of urine was reduced to numerical value after drinking for three months, as shown in Table. |
| | | | | | | | 480 | |

| | | | | | | | | |
|---|---|---|---|---|---|---|---|---|
| *1 triglyceride: normal values 50-140 mg/dl *2 obesity index: normal values -10 -+10% *3 γ-GTP: normal values less than 40 units (Adult) *4 amount of urine: normal values 500-2000 ml/day | | | | | | | | |

### [Example 8] (Cosmetics and hair restoration)

Two moles of the original solution of said bioactivator 1 was diluted with water to be 1 liter and prepare an application solution, and the resulting application solution was applied to the hair on the heads of ten subjects once a day for the test, and the number of lost hairs after each washing was counted for each subject. An average of 8 lost hairs were counted prior to said treatment, while an average of 2 lost hairs were counted one month after said application test.
Further, said application solution was applied to the face, back of the neck, and hands of a subject for testing before playing golf on a fine day in May, and very little sunburn was recognized after playing golf. Further, said bioactivator was applied to the face of the subject everyday, resulting in the number of facial spots and freckles being reduced after one month.

### [Example 9]

Two milliliters of the original solution of said bioactivator 1A was diluted with water to be 1 liter and prepare an application solution, and the resulting application solution was applied to the hair on the heads of ten subjects once a day, with the number of lost hairs after washing being counted for each subject. An average of 11 lost hairs were counted prior to treatment, while an average of 1.5 lost hairs were counted one month after said application test.

### [Example 10]

Two milliliters of the original solution of said bioactivator 2A was diluted with water to be 1 liter and prepare an application solution, and the resulting application solution was applied everyday to the faces of 5 subjects, each having remarkable number of spots and freckles, and said spots and freckles were reduced remarkably after three months.

### POSSIBILITY OF INDUSTRIAL USE

In the present invention, the oxidation of a ferric salt is prevented by the vitamins C, E, and K, and the bioactivation of said ferric salt is also reinforced by said vitamins, with said ferric salt being further stabilized by a magnesium salt, so that the effectiveness of said ferric salt is not reduced during long-term storage, the stable effectiveness of said ferric salt being invariably secured. The bioactivator of the present invention is especially useful as a therapeutic agent for treatment of serious diseases such as cancer, diabetes and the like, and as a growth promoting agent for animals and plants.

## Claims

1. A bioactivator comprising an aqueous solution containing a ferric salt, one or more kinds of vitamin selected from a group consisting of vitamins C, E, and K, said aqueous solution also containing a magnesium salt.

2. A bioactivator prepared by adding one or more kinds of vitamin selected from a group consisting of vitamins C, E, and K to an aqueous solution into which a ferric salt and magnesium salt are dissolved.

3. A bioactivator in accordance with claims 1 or 2, wherein said ferric salt and vitamin(s) are mixed in a molar ratio in the range of between 1:1 and 1:10⁶, and said ferric salt and magnesium salt are mixed in a molar ratio in the range of between 1:1 and 1:10⁶.

4. A medicine containing said bioactivator in accordance with any of claims 1 to 3.

5. A cosmetic containing said bioactivator in accordance with any of claims 1 to 3.

6. A freshness keeping agent containing said bioactivator in accordance with any of claims 1 to 3.

7. A growth promoting agent for plants and animals in accordance with any of claims 1 to 3.
